# EUROPEAN PATENT SPECIFICATION

(11) **EP 4 140 507 B1**
(45) Date of publication and mention of the grant of the patent: **17.06.2026**
(21) Application number: 21793817.4
(22) Date of filing: 25.04.2021
(51) Int. Cl.: A01N 31/02, A01N 25/06, A01N 25/34, A01P 1/00, A61L 2/18, A61L 2/22, A61L 101/34

(54) **LONG-ACTING AEROSOL CLEANER AND GERMICIDE**
LANGZEITWIRKENDER AEROSOLREINIGER UND GERMIZID
PRODUIT NETTOYANT ET GERMICIDE EN AÉROSOL À TEMPS D'ACTIVITÉ PROLONGÉ

(30) Priority: 25.04.2020 ES 202030347
(43) Date of publication of application: 01.03.2023
(73) Proprietor: Bio Logic Crop Science, S.L., 46010 Valencia (ES)
(72) Inventor: CASAÑA GINER, Victor, 46010 Valencia (ES)
(74) Representative: Soler Lerma, Santiago
(86) International application number: PCT/ES2021/070274
(87) International publication number: WO 2021/214369

(56) References cited:
- US-A- 4 129 662
- US-A- 5 167 950
- US-A1- 2009 018 213
- US-A1- 2018 220 647
- US-A1- 2019 000 086
- US-B1- 6 299 862
- NALAWADE TRIVENIMOHAN ET AL: "Bactericidal activity of propylene glycol, glycerine, polyethylene glycol 400, and polyethylene glycol 1000 against selected microorganisms", JOURNAL OF INTERNATIONAL SOCIETY OF PREVENTIVE AND COMMUNITY DENTISTRY, vol. 5, no. 2, 1 January 2015 (2015-01-01), IN, pages 114, XP093177494, ISSN: 2231-0762, DOI: 10.4103/2231-0762.155736
- MAN ADRIAN ET AL: "Effects of low-molecular weight alcohols on bacterial viability", ROMANIAN REVIEW OF LABORATORY MEDICINE, vol. 25, no. 4, 26 October 2017 (2017-10-26), pages 335 - 343, XP093177554, ISSN: 2284-5623, DOI: 10.1515/rrlm-2017-0028

## Description

The present invention is encompassed in the field of cleaning and disinfection of environments and surfaces by products in aerosol format, especially for the inactivation of viruses in the structure whereof there is a sensitive lipid membrane, such as human coronaviruses and, more specifically, covid-19. In particular, this invention addresses the effectiveness of aerosolised droplets in the peripheral area of application.

As aerosol format we refer to a receptacle that, thanks to the inclusion of a propellant with its corresponding opening and closing valve, causes the liquid formulation it contains to be expelled in the form of an aerosol, i.e. small droplets.

### Prior art

The present invention relates to a problem that is ignored in spray disinfection processes, which is the rapid evaporation of the aerosolised droplets far from the central flow of spray -peripheral area-, and its solution by means of a product in aerosol format which, thanks to its particular composition, manages to prolong the cleaning efficiency of dirt and biocide -against germs (fungi, mycobacteria, bacteria, protozoa, viruses, viroids, prions, etc.) and, in particular, human coronaviruses such as covid-19-of the peripheral sprayed liquid for a longer period of time and, in turn, does not affect its ease of application and easy drying.

In particular, it is a product that improves cleaning and disinfecting efficiency in an economical manner, addressing an innovative way to solve one of the problems that cleaning and disinfecting products in aerosol format have. The aforementioned problem is that, as they are sprayed in aerosol form and have a very small droplet size, products containing highly volatile cleaning compounds or biocides evaporate very quickly thus losing the biocidal activity necessary to kill germs (including viruses, viroids and prions).

The use of ethanol against germs is well documented. In particular, hydroalcoholic compositions against emerging bacteria and viruses, such as covid-19, for use in hand gels, or products sprayed with guns (without propellant) for surfaces are already known. Also known are aerosol products (Lysol^{®}, Sanytol^{®}) containing ethanol or isopropanol combined with water for identical uses, in addition to their uses as cleaners (removers of dirt or solid aggregates wherein germs may take refuge). There are also mixtures of short-chain alcohols (ethanol, propanol, butanol) with glycerine and/or glycols, however, such formulations have not been studied in detail in the peripheral area, nor have they apparently been optimised to solve the problem of the present invention.

Hereinafter, we will refer to products based exclusively on ethanol and propanol (included in the term n-propanol and/or isopropanol).

In fact, the choice to use only ethanol or propanol is motivated by an important reason: other biocidal products are considerably more toxic to humans and the environment. With the widespread use of aerosols for disinfection, the inhalation of quaternary ammonium compounds and as a special mention, of benzalkonium chloride or sarcosinate, which have known problems of irritation and inflammation of the respiratory system, in addition to eye problems, involuntarily occurs. The present invention solves this problem without the use of other biocides. It is noteworthy that the problem that the present invention solves is not present when there are non-volatile biocides in the peripheral spray areas. In other words, to have a good cleaning and disinfection product, either a non-volatile biocidal product is used (which would be the option that a person skilled in the art would take and, in fact, practically all the products for sale) or water is added.

However, although water is an eminently cheap compound, we have found that it does not sufficiently delay the evaporation of the peripheral areas when spraying. In addition, our solution allows using the disinfectant aerosol on electrical appliances due to the absence of water.

The peripheral spray area is an obvious concept for a person skilled in the art, but in the case of any confusion we can consider it to be the circular crown around the centre of the spray containing 5% of the sprayed product when sprayed on a surface perpendicular to the direction of the spray at about 50 cm away.

There is a sometimes overlooked aspect as regards extrapolating the biocidal efficacy of an aerosol with previously performed standardised assays. The assay times wherein the biocidal product is in contact with the germ are sometimes longer than those that actually occur on surfaces (or even air) when the product is sprayed and a peripheral area is created where there are very small separate droplets away from the central area. For example, a droplet in a normal spray range of 50 µm when deposited alone (we refer to that peripheral area after spraying) evaporates (obviously depending on its composition) in 1.5 seconds or even less in the case of alcohol with water, glycerine or other glycol components in a small percentage. However, if we take ethanol as an example, the minimum contact time to be effective against covid-19 is 5 seconds. This implies that there will be areas after spraying that do not kill the germs, thus losing effectiveness.

It is obvious that the present invention is not intended to improve the biocidal efficacy in the "central" areas of the aerosolised product, since in those areas the contact time is sufficient at a sufficient amount sprayed (greater than 5 seconds).

US 2019000086 A1 (ALIMI HOJABR et al.) 01/03/2019, paragraphs 9 and 75-83, 109, relates to antimicrobial compositions that necessarily include at least one compound containing guanide, either biguanide or biguanidine or triguanide and, on the other hand, one or more alcohols including ethanol, methanol, isopropanol or the combinations thereof. Paragraphs 75-83 disclose different types of alcohols that they use, among an extremely exhaustive list, are glycols and, therefore, propylene glycol. Modes of use are also described in paragraph 109, extensively, including the aerosol format.

However, the document makes no reference to the combination of ethanol and/or propanol combined with propylene glycol to be used in aerosol format; on the contrary, the fact of necessarily including guanide- and guanidine-type compounds, would make the person skilled in the art not try an essential element in said invention for the antimicrobial effect, in particular in aerosol format. On the other hand, all embodiments of said invention use water (as shown in all tables wherein the water content is "q.s."). This would also make the person skilled in the art think that water is a necessary component in said formulations and would not try to dispense with guanides and biguanides and, in addition, the presence of water. Neither does it make any reference to the particular benefit of the present invention, wherein the special characteristics of ethanol and isopropanol, combined with propylene glycol and in the absence of water, make an aerosol application particularly effective. It is an indisputable fact that water has a higher volatility than that of propylene glycol, and that the presence of propylene glycol in US 2019000086 A1 and at the same time the absence of water, is not contemplated in said patent, nor is there any indication that the person skilled in the art would deviate from the teachings of US 2019000086 A1 if he/she intends to apply that knowledge in an aerosol format. The presence of additional antimicrobial compounds necessary according to said document, which are indeed solid at room temperature and remain on the surfaces once dry, wouldn't even lead one to think about a problem in the contact time to have a suitable biocidal effect.

US 2018220647 A1 (BINGHAM JAMES EDMUND et al.) 08/09/2018, paragraphs 15-18, discloses disinfectant compositions for surfaces including C1-C6 alkanols, and mixtures thereof. As can be seen from paragraph 16, however, all of these compositions contain water. In the most extreme case, it mentions an alcohol content of 98%, the rest being understood to be water. Therefore, the present invention, unlike and in view of US 2018220647 A1, takes a completely different path by substantially removing the water content from the formulation (all industrial alcohol contains a percentage of residual water, but there are those with content lower than 1% in water for special applications). US 2018220647 A1 also discloses the addition of peracids, of known microbial action due to their oxidising properties implicit in the additional oxygen of the peracid group. These combinations have an additional problem as we have verified. The oxidation of peracids in tin aerosol containers means that the tin aerosol cans have greatly increased corrosion, and hence, they are not safe for sale to the general public. They can generate anything from leaks of content due to corrosion of the tin, of the valve gaskets (also applicable to aerosol containers with aluminium cans), and of the dome and of the base of the can, right up to the explosion of the can itself as we have verified in laboratory assays where the formulation contained 70% ethanol, 29% water, 0.845% peroxypyruvic acid and adjusted with 12 M HCl at a pH of 1.5. The explosion of the tin can thus formulated (0.18 mm thick, Golden lack epoxy) spontaneously occurred 4 months after formulation being kept in an ATEX cabinet to observe stability at room temperature. It is evident that not only the teaching of US 2018220647 A1 is not applicable to the scope of this patent restricted to aerosols. Furthermore, US 2018220647 A1 does not lead the person skilled in the art to dispense with the peracids described as microbial agents, nor does it lead to choosing propylene glycol as a compound of the formulation that replaces (up to the industrially acceptable level) the presence of water.

US 2009018213 A1 (SNYDER MARCIA et al.) 15/01/2009, paragraphs 10-14, 17, 23, 24, 80 relates to the use of C1-C6 alkanol formulations with at least one compound having virucidal activity, such as cationic oligomers and polymers, chaotropic agents and mixtures thereof. It also discloses topical virucidal effect in mammals. In paragraph 13, it mentions a foaming surfactant, which is not the case with the compound propylene glycol. In paragraph 17, it mentions the use in aerosols, however our invention precisely avoids the use of these cationic and chaotropic agents because they increase the toxicity of the compounds that are emitted in aerosol format by non-professional personnel, since exposure by inhalation in the type of use contemplated in the present patent is very different from the exposure by topical application on surfaces or even in the air of a room. Paragraph 23 relates to mixtures of C1-C6 alkanols, without mentioning propylene glycol. Finally, paragraph 80 relates to aerosolisation, but in fact all the formulations contemplated in US 2009018213 A1 suffer from the problem that the effect of the alkanols is so short that they do not have an optimal surface disinfection time, as they lack the propylene glycol described in the present patent, and, in addition, have a higher inhalation toxicity due to the use of the named cationic compounds.

On the other hand, the use of propylene glycol in the state of the art is related to the decrease in surface tension and extensibility of the product, while that property is just the opposite of what is intended with the solution provided here: we have observed that it is much more relevant for an aerosol that the biocidal droplet remains sufficient time on the surface than the effect of greater evaporation caused by the fact that it serves to spread out.

Having considered these documents as the closest prior art, we see that in all three cases, additional compounds as well as C1-C6 alkanols are used to improve microbiocidal efficacy. In other words, viewed as a whole, it is not considered that an aerosol, essentially without water, for aerosol use as a surface disinfectant, such as that of the present invention, is sufficient for the intended purpose. In US 2019000086 A1, paragraph 81, the use of propylene glycol is mentioned, but not as a compound that may benefit the biocidal action, but as a simple vehicle that can be used to help it be distributed on the skin, to the same degree that an alcohol is used as a vehicle to give more volume to the formulations of, for example, repellents, and to be able to distribute the repellent active ingredient over more areas of the skin. The fundamental thing in this case is that the present invention excludes the use of water, which seems to be necessary according to all the examples of embodiment of said patent, and, in addition, excludes the use of additional biocides such as those that each of the three previous documents claim.

US 6 299 862 B1 describes a rapidly drying aerosol composition intended for sanitising personal used surfaces such toilet seats. The spray has a n average drying time of 12-15 seconds. The aerosol composition comprises 52-75 wt.% of ethanol and/or isopropanol and 25-48 wt.% of a hydrocarbon propellant blend essentially consisting of 85 wt. % isobutane and 15 wt.% propane.

The solution adopted to solve this problem is based on:
A cleaning and disinfection product characterised in that:
∘ **It has** the format of an aerosol product.
∘ The propellant gas is liquefied petroleum gas, since nitrogen or CO2 do not allow achieving a spray throughout the life of the appropriate product for the purposes of covering the largest possible surface homogeneously and modulating the contact time of the peripheral area of the spray.
∘ The valve allows a discharge rate of 0.2 g/s to 4 g/s, as quantities less than 0.2 g do not facilitate the rapid application of the product over a large surface area and those greater than 4 g/s generate too irregular a distribution of the product on the surface or considerably wet the floor if it is sprayed in the air.
∘ The composition of the liquid contained in the container, with the exception of the propellant, when activating the valve consists of in percentage by weight:
   ▪ Ethanol and/or propanol between 60% and 90% so that biocidal activity is ensured. Preferably, the propanol is isopropanol.
   ▪ Propylene glycol up to 100%, since other glycols either evaporate too quickly, or evaporate too slowly and are detrimental to need that the product is applied, disinfects and a consumer feeling of cleanliness free from residues that take time to dry.
   ▪ Optionally, a fragrance up to 5% in which case the propylene glycol content is conveniently reduced to reach 100%, preferably between 0.1% and 1%.
∘ The amount in percentage by weight of propellant gas within the package with respect to 100% of the weight of the package interior is greater than 10%, preferably at least 30% otherwise, the optimal pressure for a product distribution is too low. The optimal range is between 30% and 90%.

The product of the present invention has as the only compound with recognised biocidal activity in accordance with the guidelines of the Food and Drug Administration of the United States (FDA USA) is ethanol and lacks any solid biocidal compound at room temperature except for thymol and menthol as part of the fragrance. At this point we would like to emphasize that we should not confuse the fact that the fragrance is also biocidal to a certain degree per se. Thymol is recognised by the FDA USA as an active biocide against human coronavirus, in the same way, we observe similar or superior biocidal activity of eucalyptol, when used in a concentration greater than 0.03% in the sprayed liquid.

The fragrance contains at least one compound chosen from: thymol, linalool, citral, menthol, lavandin, geraniol, eucalyptol.

The average particle size of the aerosol it creates is between 5 µm and 100 µm, since outside these ranges, the optimal balance between cleaning, disinfection and product drying is lost. In this regard, it is also convenient that at least 10% of droplets in the aerosol that the product creates have a particle size of less than 50 µm.

In addition to the product per se, all possible combinations of the aforementioned can be combined to have an adequate cleaning and disinfection process.

The use of propylene glycol combined exclusively with ethanol and/or propanol and a fragrance as a disinfecting agent and cleaner of air and surfaces to prolong the contact time of ethanol and/or propanol with germs or dirt, for a propylene glycol concentration with respect to the total of the liquid of at least 10%, preferably 20%, more preferably of at least 28%, is something novel and that solves the problem posed in an effective way without being detrimental to other parameters such as hazardous nature of the product with electrical devices (as it has no water), drying time, form of distribution of droplet sizes, and other characteristics already mentioned.

According to the present description use does mean a non-therapeutic use, if the surface is a human or animal body part.

Additionally, after stability studies with tin cans, the formulations belonging to the state of the art containing water have a stability much lower than the stability of the formulations containing propylene glycol and mixtures of ethanol and propanol (in any ratio between the two alcohols of 0% to 100% excluding propylene glycol).

### Example 1, according to the invention:

A formulation is prepared containing by weight:
65% propylene glycol, technical grade
35% completely denatured ethanol, technical grade (<1% water)

It is contained in a transparent PET can to be able to observe the separation of phases, adding 50% by weight of the biocidal mixture and 50% of liquefied petroleum gas.

It is also contained in a tin can of 0.18 mm thickness and 750 mL capacity (inner epoxy Gold resin supplied by Colep, Portugal) to be able to observe the stability to corrosion over time. (6 samples)

### Example 2, not according to the present invention

Formulation according to Example 43, Table 13 of US 2009018213 A1
21.88% water
74.1% ethanol
1.88% polyquaternium-2 Acid
1.6% PEG-10 dimethicone
0.54% PEG-12 dimethicone

It is contained in a transparent PET can to be able to observe the separation of phases, adding 50% by weight of the biocidal mixture and 50% of liquefied petroleum gas.

It is also contained in a tin can of 0.18 mm thickness and 750 mL capacity (inner epoxy Gold resin supplied by Colep, Portugal) to be able to observe the stability to corrosion over time. (6 samples)

### Example 3, not according to the present invention

60% water
5% propylene glycol, technical grade
35% completely denatured ethanol, technical grade (<1% water)

It is contained in a transparent PET can to be able to observe the separation of phases, adding 50% by weight of the biocidal mixture and 50% of liquefied petroleum gas.

It is also contained in a tin can of 0.18 mm thickness and 750 mL capacity (inner epoxy Gold resin, supplied by Colep, Portugal) to be able to observe the stability to corrosion over time. (6 samples). In this case, a valve with a very small discharge rate was selected to prevent Petri dishes from over-flooding in the activity assay (discharge rate 0.05 g/s).

For distribution and residence assays of biocidal liquid, 5 adjacent Petri dishes (90 mm x 14.2 mm) are arranged in a line, previously inoculated with a Candida sp. own culture from previous assays of plastic polymer decomposition experiments, and with standard potato-agar-dextrose culture medium, and the central dish is sprayed for one second. They are left covered, allowed to dry and put in the stove at 37°C for 7 days. Additionally, two control dishes are placed at the ends, one on each side of the central point of the assay.

Experiments performed with the 3 examples.
1. Observation of phase homogeneity in PET aerosol can
   Example 1 shows after stirring that the phases are mixed and remain stable.
   Example 2 shows after stirring that the phases are partially mixed and the formulation is stable but after 18 days of observation at room temperature a certain separation of water is seen at the bottom of the can.
   Example 3 shows after stirring that the phases do not mix and there is a clear difference between the liquefied gas phase of the oil and the water phase which appears completely separated in approximately the end quarter of the can.
2. Observation of the stability of tin cans in a 50°C oven for 3 months
   Example 1 shows that the 6 cans remain without any leaks after the 3 month stability period.
   Example 2 shows after one month and one week, 2 cans have leaks in the bottom seam (triple point) of the can. In the two month review, the remaining four cans remain stable; however, after two months and two weeks the remaining four cans are all leaking. The probable reason is the need for a greater epoxy resin coating due to the presence of water and, in addition, the possible effect on the corrosion of polyquaternium-2 acid.
   Example 3 shows after one month that all the cans have leaked, showing a high degree of corrosion at the triple point of the can.
3. Observation of antimicrobial capacity
   Example 1 shows that the central dish and its two adjacent dishes did not show growth of Candida sp. yeasts, while in the two dishes furthest from the central dish - except for the controls - the growth occurred only partially at the ends furthest from the central point of the experiment. Control dishes showed considerable growth of Candida sp.
   Example 2 shows that the central dish and its two adjacent ones did not show growth of Candida sp. yeasts, while in the two dishes furthest from the central dish - except the controls - the growth occurred only partially at the ends furthest from the central point of the experiment, as in Example 1. Control dishes showed considerable growth of Candida sp.
   Example 3 shows that the central dish did not show yeast growth Candida sp., while in the two adjacent dishes isolated colonies could be seen, unlike the two previous examples. In the two dishes furthest from the central one - except for the controls - growth occurred to a greater extent than in Examples 1 and 2.

This, we believe, has an explanation that corresponds to the object of the present invention: in the place of the spraying, the liquid concentration of the three examples is sufficient to eliminate all the yeasts. In the two adjacent dishes, the evaporation time is already very relevant, if it has water it does not have so much time to cover the whole dish because the ethanol evaporates too quickly. And that effect is seen more in the furthest away dishes (excluding controls).

The growth in the dishes treated in Examples 1 and 2 is very probably due to the fact that the spray cone did not completely cover all the dishes.

The effect provided by propylene glycol (longer evaporation time) is not relevant compared to Example 2, since it carries biocide that does not evaporate and are solid at room temperature.

What is gathered from the assay is that the formulations according to our invention allow a longer contact time compared to those that, instead of propylene glycol, carry water, increasing the biocidal capacity. On the other hand, for aerosols in tin cans, our invention allows a greater durability of the can than when water and/or compounds with corrosion capacity, especially ionic that accelerate the oxidation/reduction processes of the tin metal, are added. In addition, the absence of solid elements in the aerosol biocidal compositions object of the present invention avoids the problem of possible allergies to ionic biocides that may exist when inhaled in excess.

## Claims

1. Cleaning and disinfection product **characterised in that**:
a. It has the format of an aerosol product.
b. The propellant gas is liquefied petroleum gas.
c. The valve allows a discharge rate of 0.2 g/s to 4 g/s.
d. The composition of the liquid contained in the container, with the exception of the propellant, when activating the valve consists of in percentage by weight:
i. Ethanol and/or propanol between 60% and 90%.
ii. Propylene glycol up to 100%.
iii. Optionally, a fragrance up to 5%, in which case the propylene glycol content is conveniently reduced to reach 100%.
e. The amount in percentage by weight of propellant gas within the container relative to 100% of the weight of the interior of the container is greater than 10%, preferably at least 30%.

2. Cleaning and disinfection product according to claim 1, further **characterised in that** the product lacks any solid biocidal compound at room temperature except for thymol and menthol as part of the fragrance.

3. Cleaning and disinfection product according to claim 1, **characterised in that**:
a. The fragrance contains at least one compound chosen from: thymol, linalool, citral, menthol, lavandin, geraniol, eucalyptol.

4. Cleaning and disinfection product according to claim 1, **characterised in that** the average particle size of the aerosol it creates is between 5 µm and 100 µm.

5. Cleaning and disinfection product according to claim 1, **characterised in that** there are at least 10% of droplets in the aerosol that it creates with a particle size of less than 50 µm.

6. Non-therapeutic surface disinfection process **characterised in that** it uses an aerosol product according to claim 1.

7. The non-therapeutic use of propylene glycol combined exclusively with ethanol and/or propanol and a fragrance as a disinfecting agent and air and surface cleaner to prolong the contact time of ethanol and/or propanol with germs or dirt, for a concentration of propylene glycol with respect to the total liquid of at least 10%, preferably of at least 28%.

8. Cleaning and disinfection product according to any of claims 1 to 5 or use according to claim 7, wherein the propanol is isopropanol.

## Patentansprüche

1. Reinigungs- und Desinfektionsprodukt, durch Folgendes gekennzeichnet:
a. Es weist das Format eines Aerosolprodukts auf.
b. Das Treibgas ist Flüssiggas.
c. Das Ventil ermöglicht eine Abgaberate von 0,2 g/s bis 4 g/s.
d. Die Zusammensetzung der im Behälter enthaltenen Flüssigkeit, mit Ausnahme des Treibgases, besteht bei Aktivierung des Ventils in Gewichtsprozent aus:
i. Ethanol und/oder Propanol zwischen 60 % und 90 %.
ii. Propylenglykol bis zu 100 %.
iii. Optional einem Duftstoff von bis zu 5 %, wobei der Propylenglykolgehalt in geeigneter Weise reduziert wird, um 100 % zu erreichen.
e. Der Treibgasgehalt in Gewichtsprozent innerhalb des Behälters, bezogen auf 100 Gew.-% im Inneren des Behälters, ist höher als 10 %, vorzugsweise mindestens 30 %.

2. Reinigungs- und Desinfektionsprodukt nach Anspruch 1, ferner **dadurch gekennzeichnet, dass** das Produkt bei Raumtemperatur keine festen bioziden Verbindungen enthält, mit Ausnahme von Thymol und Menthol als Bestandteil des Duftstoffs.

3. Reinigungs- und Desinfektionsprodukt nach Anspruch 1, durch Folgendes gekennzeichnet:
a. Der Duftstoff enthält mindestens eine Verbindung, ausgewählt aus: Thymol, Linalool, Citral, Menthol, Lavandin, Geraniol, Eukalyptol.

4. Reinigungs- und Desinfektionsprodukt nach Anspruch 1, **dadurch gekennzeichnet, dass** die mittlere Partikelgröße des erzeugten Aerosols zwischen 5 µm und 100 µm liegt.

5. Reinigungs- und Desinfektionsprodukt nach Anspruch 1, **dadurch gekennzeichnet, dass** in dem von ihm erzeugten Aerosol mindestens 10 % Tröpfchen mit einer Partikelgröße von weniger als 50 µm vorhanden sind.

6. Nichttherapeutisches Oberflächendesinfektionsverfahren, **dadurch gekennzeichnet, dass** es ein Aerosolprodukt nach Anspruch 1 verwendet.

7. Nichttherapeutische Verwendung von Propylenglykol in Kombination ausschließlich mit Ethanol und/oder Propanol und einem Duftstoff als Desinfektionsmittel und Luft- und Oberflächenreiniger zur Verlängerung der Kontaktzeit von Ethanol und/oder Propanol mit Keimen oder Schmutz bei einer Konzentration von Propylenglykol in Bezug auf die Gesamtflüssigkeit von mindestens 10 %, vorzugsweise von mindestens 28 %.

8. Reinigungs- und Desinfektionsprodukt nach einem der Ansprüche 1 bis 5 oder Verwendung nach Anspruch 7, wobei das Propanol Isopropanol ist.

## Revendications

1. Produit de nettoyage et de désinfection **caractérisé en ce que** :
a. Il a le format d'un produit aérosol.
b. Le gaz propulseur est du gaz de pétrole liquéfié.
c. La vanne permet un débit de décharge de 0,2 g/s à 4 g/s.
d. La composition du liquide contenu dans le récipient, à l'exception du propulseur, lors de l'activation de la vanne, comprend en pourcentage en poids :
i. Entre 60 % et 90 % d'éthanol et/ou de propanol.
ii. Jusqu'à 100 % de propylène glycol.
iii. Facultativement, jusqu'à 5 % d'un parfum, auquel cas la teneur en propylène glycol est opportunément réduite pour atteindre 100 %.
e. La quantité en pourcentage en poids de gaz propulseur à l'intérieur du récipient par rapport à 100 % du poids de l'intérieur du récipient est supérieure à 10 %, de préférence d'au moins 30 %.

2. Produit de nettoyage et de désinfection selon la revendication 1, **caractérisé en outre en ce que** le produit est dépourvu de tout composé biocide solide à température ambiante, à l'exception du thymol et du menthol dans le cadre du parfum.

3. Produit de nettoyage et de désinfection selon la revendication 1, **caractérisé en ce que** :
a. Le parfum contient au moins un composé choisi parmi : le thymol, le linalol, le citral, le menthol, le lavandin, le géraniol, l'eucalyptol.

4. Produit de nettoyage et de désinfection selon la revendication 1, **caractérisé en ce que** la taille moyenne des particules de l'aérosol qu'il crée est comprise entre 5 µm et 100 µm.

5. Produit de nettoyage et de désinfection selon la revendication 1, **caractérisé en ce qu'**il y a au moins 10 % de gouttelettes dans l'aérosol qu'il crée avec une taille de particules inférieure à 50 µm.

6. Procédé de désinfection de surface non thérapeutique **caractérisé en ce qu'**il utilise un produit aérosol selon la revendication 1.

7. Utilisation non thérapeutique de propylène glycol combiné exclusivement avec de l'éthanol et/ou du propanol et un parfum comme agent désinfectant et nettoyant d'air et de surface pour prolonger le temps de contact de l'éthanol et/ou du propanol avec des germes ou des saletés, pour une concentration de propylène glycol par rapport au liquide total d'au moins 10 %, de préférence d'au moins 28 %.

8. Produit de nettoyage et de désinfection selon l'une quelconque des revendications 1 à 5 ou utilisation selon la revendication 7, le propanol étant l'isopropanol.
